# EUROPEAN PATENT APPLICATION

(11) **EP 1 129 681 A1**
(43) Date of publication of application: **05.09.2001**
(21) Application number: 01830128.3
(22) Date of filing: 23.02.2001
(51) Int. Cl.: A61F 9/007

(54) **Phacoemulsification tip**

(30) Priority: 01.03.2000 IT RM000038
(71) Applicant: Optikon 2000 S.p.a., 00138 Roma (IT)
(72) Inventor: Pezzola, Alessandro, 25128 Brescia (IT)
(74) Representative: Iannone, Carlo Luigi

(57) **Abstract**

The invention concerns to a phacoemulsification tip (1) having one end with cutting front section (2), a central channel (4) for the passage of the removed tissue, characterized in that at short distance from the cutting end (2) a diaphragm (3) is provided, reducing the inner diameter of the channel of the tip (1) of some tenth of millimeters of its length.

## Description

The present invention relates to a phacoemulsification tip.

More specifically, the invention concerns a tip of the above kind, made up of titanium, particularly studied to use high vacuum levels.

It is known that the traditional phacoemulsification tips must be used with a relatively low vacuum level to avoid the risk of collapse of the front chamber of the eye.

This limits the possibility of keeping the nucleus during its emulsification and to efficiently suck it. In fact, while sucking tends to draw the material toward the tip, the ultrasonic vibration tends to separate the same.

It is well evident the need of having available a solution allowing to use under safety conditions higher vacuum levels. To this end, it is indispensable to know the mechanical of the phenomenon bringing to the collapse of the front chamber.

In a phacoemulsification system employing sucking by a peristaltic pump, under not occluded tip condition, the vacuum level present in the sucking line is very low. In fact, the peristaltic pump produces a low liquid flow (usually a flow of 18 - 30 cc/min), and the phacoemulsification tip has a relatively large inner diameter (about 0.9 mm) and a limited length (lower than 30 mm). Under these conditions, impedance offered to the passage of the liquids by the tip, and consequently the vacuum along the line, is very limited (about 30 - 40 mmHg).

The saline solution flow, coming from the infusion bottle, is widely sufficient to maintain the intraocular pressure and to ensure the stability of the front chamber.

When a fragment of the nucleus is taken, the tip occlude, and the vacuum along the sucking line rises up to the limit selected by the surgeon.

Growing of the vacuum creates a storage of potential energy by the elastic components presents in the circuit (walls of the tube, air bubbles, etc,) that will be deformed.

When the surgeon activates the emission of ultrasounds and emulsifies the cataract, the occlusion is removed, and the elastic component of the circuit take again the original shape, instantaneously freeing the stored potential energy: in order to have the vacuum immediately to the values preceding the occlusion, a liquid flow is established at the outlet of the front chamber, limited only by the impedance of the sucking tip.

If the selected vacuum is high, the flow from the irrigation bottle is not sufficient to balance the sucking flow peak and a collapse of the front chamber of the eye occurs.

It is evident that to maintain the stability of the front chamber, it is necessary to find a system making that the potential energy stored during the occlusion is gradually and not instantaneously released, so as to limit the amplitude of the flow peak (increasing its duration) up to values that can be easily balanced by the irrigation flow.

A simple way to obtain this result is to reduce the inner diameter of the sucking tip in such a way to remarkably increase its impedance to the passage of the fluids: in fact, said impedance is inversely proportional to the square of the diameter, thus a reduction of said diameter in the order of 20% causes an increase of the impedance of 40%.

Phacoemulsification tips having the above mentioned features are already available on the market.

However, also the force keeping the nucleus pieces stuck to the end of the titanium tip is directly proportional to the front surface of the same (F=VAC x S), and therefore to the square of the tip diameter.

For this reason, in this kind of tips, possibility of increasing the vacuum level with respect to the one used with standard tips does not produce any real advantage as far as the capability of firmly keeping the nucleus is concerned.

In fact, if the reduction of the inner diameter allows to increase the vacuum level of 40%, for example between 200 and 280 mmHg, the same it is obtained the same mass holding force that would be obtained in a tip having standard dimensions with the original vacuum level (200 mmHg).

Another possible known solution (Cobra tip, Surgical Design) provides the realization of a phacoemulsification tip having a standard front diameter (I.D. = 0.9 mm), and wherein, at a distance of 1-2 mm from the end of the diameter reduces at 0.5- 0.6 mm.

In this way, the section in contact with the cataract has standard dimensions and thus can exert a good nucleus holding force, the subsequent reduction of the diameter allows to use higher vacuum levels.

However, problems exist limiting the use of said tip, and particularly the need of using rigid sleeves (not made up of silicone), which are not well accepted by most surgeons, and the fact that, particularly if ultrasounds are used according to a pulsed mode, some cataract particles can stop in the little diameter zone of the tip, inducing the occlusion of the same.

In U.S. - A - 5.112.339 (Zelman) it is described an apparatus to extract cataract tissue. In said patent the above mentioned problems are explained.

In view of the above, it is well evident the need of having at disposal a phacoemulsification tip as the one suggested according to the present invention that allows the use of high vacuum levels, thus avoiding the above mentioned drawbacks.

It is therefore specific object of the present invention a phacoemulsification tip having one end with cutting front section, a central channel for the passage of the removed tissue, characterized in that at short distance from the cutting end a diaphragm is provided, reducing the inner diameter of the channel of the tip of some tenth of millimeters of its length.

Preferably, said diaphragm has an asymmetrical shape, particularly having a substantially vertical wall (or a wall substantially vertical with respect to the tip axis) on the side faced toward the cutting end, and a conical shape on the side faced toward the base of the same tip.

According to the invention, said diaphragm can be provided at about 1.8 mm from the cutting end of the tip, reducing the inner diameter of the channel at about 0.5 mm.

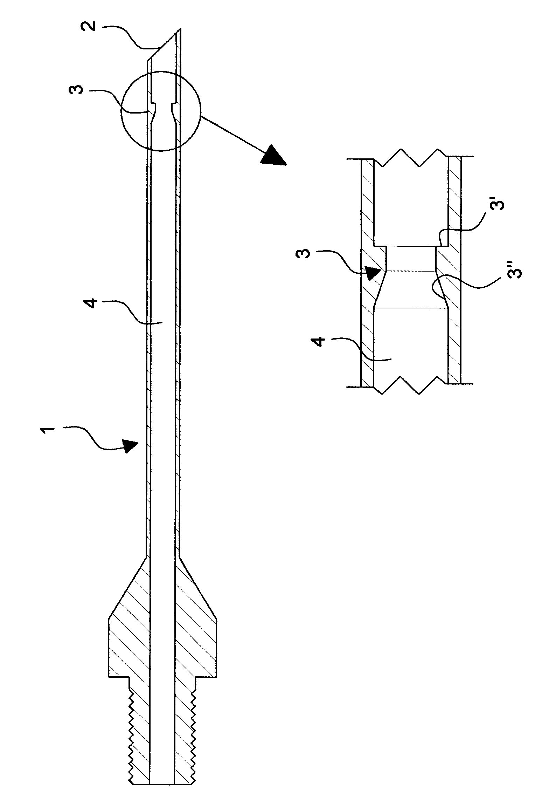

The present invention will be now described, for illustrative but not limitative purposes, according to its preferred embodiments, with particular reference to the figure of the enclosed drawing, wherein it is shown a section view of the phacoemulsification tip.

Point 1 according to the invention has outer dimensions identical to those of a standard tip, but an inner geometry suitably studied to slow the motion of the cataract and of the fluids sucked at the moment of breaking of the occlusion.

As it can be seen from the figure, the front section of tip 1 is identical to the one of a standard tip, thus maintaining, under the same vacuum conditions, the mass holding force.

At short distance from the end 2 (about 1.8 mm) it has been obtained a diaphragm 3, within the central channel 4 of the tip 1, reducing the diameter of the tip, particularly at 0.5 mm for some tenth of millimeters of its length.

Said diaphragm 3 has an asymmetrical shape: a substantially vertical wall 3' (perpendicular to the axis of the tip 1) of the side faced toward the end 2 of the tip 1, and a conical shape 3" on the side faced toward the base of the same tip 1.

Tip 1 according to the invention operates according to the following mechanism

The front section of dimensions corresponding to those of a standard tip allows to have a good ratio between the selected vacuum limit and the nucleus holding force.

The substantial reduction of the diameter remarkably increases the hydraulic impedance of the tip, thus limiting flow peaks during the removal phase and thus allowing to set higher vacuum values. Since the narrowing zone is short and localized in a zone where it is strong the oscillation of the tip 1, and thus the emulsification action of the nucleus, the clogging phenomenon mentioned with reference to the "cobra" tip are not possible.

On the contrary, under this point of view, since the nucleus is more finely emulsified (having also to pass through the diaphragm), the tip 1 according to the invention is less subjected to clogging problems than the standard tips.

Front wall 3' (vertical) of the diaphragm 3, axially oscillating with the tip 1, reduces the ultrasound emission phase, inducing an additional emission surface of the ultrasounds, thus improving the nucleus emulsification. Said turbulence zone is within the tip 1 thus not disturbing the eye structures.

Asymmetry of the diaphragm 3 induces a mechanical pumping action during the ultrasonic oscillation, which is more sensitive on the solid particles of the nucleus, slowing down the passage of the same particles through the diaphragm 3, further reducing the flow sucking peaks, and thus the collapse possibility of the front chamber at high vacuum levels.

### TESTS

It has been carried out a series of measurements employing a diaphragm, and comparing the same with a standard tip.

The obtained results are really interesting.

The object of the tests was that of measuring the resistance to the fluid passage with different vacuum levels and to verify the effect of the turbulence produced by the ultrasounds on the flow peaks.

Testing setup was the following:
- Venturi pump with a vacuum level set up system;
- Graduated test tube to measure the flow sucked through the tip/handle with different vacuum levels;
- Tip having a 0.5 mm diaphragm;
- Standard tip.

The obtained results are the following:

| Vacuum | Flow (cc/min) | Flow (cc/min) | |
|---|---|---|---|
| | Standard tip | Tip with diaphragm | Tip with diaphragm |
| | Without U/S | Without U/S | With U/S |
| 100 | 86 | 50 | 36 |
| 150 | 112 | 60 | 42 |
| 200 | 130 | 72 | 50 |
| 250 | 147 | 83 | 57 |
| 300 | 160 | 96 | 62 |
| 350 | 170 | 103 | 68 |
| 400 | 176 | 110 | 76 |

The first column indicates the vacuum level set up on the Venturi;
the second one is the flow passing through a standard tip with that vacuum level;
the third one is the tip flow through the diaphragm tip without activating the ultrasounds (it only refers to the hydraulic effect of the neck);
the fourth one is the flow through the diaphragm tip with the ultrasounds active at 100 micron.

It has also been tried to activate the ultrasounds and to measure the flow with the standard tip: the flow increase of about 5 - 10 cc/min with the U/S active. Substantially, the flow peaks obtained with a standard tip with a vacuum of 150 mmHg are obtained in the diaphragm tip with a vacuum of 400 mmHg.

If it is taken into consideration that to break the cataract and to produce the sucking it is necessary that the ultrasounds are active, it is noted that the diaphragm tip, with power set at 100 micron, produces flow peaks at 400 mmHg, obtained at 80 - 85 mmHg with the standard tip.

With lower power levels, the beneficial "effect" on the flow of the U/S would be lower, but already at 50 micron it is of about 16 - 18 cc/min.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Phacoemulsification tip having one end with cutting front section, a central channel for the passage of the removed tissue, **characterized in that** at short distance from the cutting end a diaphragm is provided, reducing the inner diameter of the channel of the tip of some tenth of millimeters of its length.

2. Phacoemulsification tip according to claim 1, **characterized in that** said diaphragm has an asymmetrical shape.

3. Phacoemulsification tip according to claim 2, **characterized in that**, said diaphragm has a substantially vertical wall (or a wall substantially vertical with respect to the tip axis) on the side faced toward the cutting end, and a conical shape on the side faced toward the base of the same tip.

4. Phacoemulsification tip according to one of the preceding claims, **characterized in that** said diaphragm is provided at about 1.8 mm from the cutting end of the tip, reducing the inner diameter of the channel at about 0.5 mm.

5. Phacoemulsification tip according to one of the preceding claims, substantially as illustrated and described.
